Europäisches Patentamt

European Patent Office

Office européen des brevets

(11) Publication number: **0 456 215 A1**

## EUROPEAN PATENT APPLICATION

(21) Application number: 91107488.8

(22) Date of filing: 08.05.91

(51) Int. Cl.⁵: **C12P 21/08**, G01N 33/577, G01N 33/576

(30) Priority: 11.05.90 US 522286

(43) Date of publication of application:
**13.11.91 Bulletin 91/46**

(84) Designated Contracting States:
**AT BE CH DE ES FR GB IT LI NL**

(71) Applicant: **ABBOTT LABORATORIES**
**CHAD-0377, AP6D/2, One Abbott Park Road**
**Abbott Park, Illinois 60064-3500(US)**

(72) Inventor: **Mimms, Larry T.**
**8 Shoshoni Trail**
**Lake Villa, Illinois 60046(US)**
Inventor: **Floreani, Marco F.**
**320 Douglas Avenue**
**Wauekgan, Illinois 60085(US)**

(74) Representative: **Modiano, Guido et al**
**MODIANO, JOSIF, PISANTY & STAUB**
**Modiano & Associati Via Meravigli, 16**
**I-20123 Milano(IT)**

(54) Monoclonal antibodies to pres2 and pres1 polypeptides of the hepatitis B viral envelope.

(57) Murine monoclonal antibodies are provided which bind specifically to the PreS1 and PreS2 polypeptides of the hepatitis B virus surface antigen (HBsAg) complex and which are useful to assay for PreS1 or PreS2 in samples of physiological material. The antibodies can also be used to subtype HBV, to raise the immunogenicity of blood plasma against HBV, to measure anti-PreS1 and anti-PreS2 antibodies, and to identify and characterize antigenic epitopes of PreS1 and PreS2 which may be useful in HBV subunit vaccines.

EP 0 456 215 A1

Background of the Invention

Hepatitis B virus (HBV) infections represent a major public health problem throughout the world. Vaccination is the most efficient way to prevent acute hepatitis B infection and its chronic sequelae, e.g., liver cirrhosis and hepatoma. A hepatitis B vaccine used with good success consists of viral surface antigen (HBsAg) which is isolated from the blood of chronic virus carriers as noninfectious 22 nm particles. The 22 nm particles primarily contain a major protein of 24,000 d (p24) and its glycosylated form, gp27. Recently introduced vaccines comprise p24 which is synthesized by yeast cells that have been transfected with HBV gene S, which codes for p24. The safety and efficacy of these vaccines have been established but their immunogenicity is insufficient in a certain proportion of recipients. Thus, a need exists for more immunogenic HBV vaccines, the identification of which will require a more advanced knowledge of the location, structure, and immunogenicity of antigenic sites on HBV proteins.

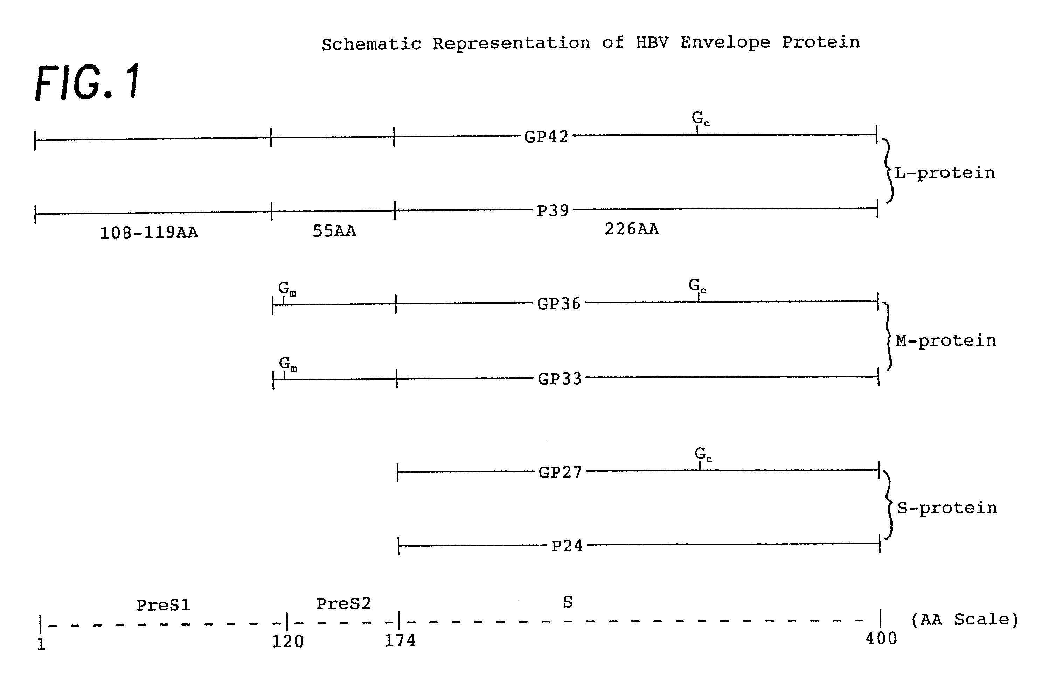

The protein composition of the HBV envelope has been determined and mapped within the viral genome. Figure 1 summarizes a model proposed by N. Herrmann et al., J. Virol., 52, 396 (1984). In this model, the three HBV envelope proteins and their glycosylated forms are derived from one continuous open reading frame (ORF), which is divided into the S, PreS2, and PreS1 regions. The S gene starts at the third or fourth start codon of this ORF, depending on the viral subtype, and codes for the major HBV envelope protein, the S protein, which includes p24 and its glycosylated form gp27. The PreS region contains two additional initiation sites in the same reading frame with the S gene allowing the expression of two larger hepatitis B surface antigen molecules: M protein (consisting of the 55 amino acids of PreS2 + S) and L protein (consisting of 108 or 119 amino acids of the PreS1 + PreS2 + S protein). M protein consists of singly or doubly glycosylated forms, gp33 and gp36, respectively. L protein consists of an unglycosylated and a singly glycosylated form, p39 and gp41, respectively.

In the blood of HBV infected persons, only a very small portion of the total hepatitis B surface antigen exists in complete virions (the Dane particles). Two other morphological forms, 22 nm spherical particles and filaments 22 nm in diameter and of variable length, lack capsid or DNA and are produced in an excess which may be as high as $1 \times 10^6$:1 over the 42 nm "Dane" particles. The protein composition of these morphological forms differs considerably. Up to 20% of total envelope protein in HBV Dane particles is L protein compared to only about 1% of total protein in the 22 nm spherical particles.

Overproduction of L protein in transfected cells leads to inhibition of secretion of L protein particles from these cells. It has been hypothesized that the L protein may play an essential role in the secretion and morphogenesis of complete virions.

The PreS1 regions of L protein may contain a specific receptor for the human hepatocytes as indicated by its ability to bind to Hep G2 cells (A. R. Neurath et al., Cell, 46, 429 (1986)). Specific binding of the PreS2 region to polymerized human serum albumin has also been proposed as a possible mechanism for association of HBV with the hepatocyte plasma membrane (M. Imai et al., Gastroenterology, 76, 242 (1979)-).

Regardless of the role of PreS-containing proteins in viral structure or function, PreS1 and PreS2 proteins have been shown to be highly immunogenic in mice and their combination with S protein may potentiate the immune response to the S protein (D. R. Milich et al., Science, 228, 1195 (1985)). A synthetic peptide vaccine containing only a portion of the PreS2 region elicited an immune response to PreS2 and protected the inoculated chimpanzees from HBV infections after challenge with virus (A. Neurath et al., Vaccine, 4, 35 (1986)). The available hepatitis B vaccines differ significantly in their PreS composition, and the relative immunogenicity of PreS-containing vaccines in humans remains to be elucidated.

Ausria[R]II, Auszyme[R]II and Monoclonal Auszyme[R] are commercially available assays for the detection of hepatitis B surface antigen (HBsAg) (Abbott Laboratories, N. Chicago, IL). Monoclonal Auszyme uses only monoclonal antibodies to protein S. These HBsAg assays do not allow discrimination between L, M, and S proteins.

A commercially-available direct solid-phase radio-immunoassay (RIA) for anti-HBs detection and quantitation has been available since 1975 (AUSAB, Abbott Laboratories) (I. K. Mushahwar et al., J. Med. Virol., 2, 77 (1978)). In this assay, the serum or plasma specimen is incubated with a solid-phase (polystyrene bead) that has been coated with human plasma-derived hepatitis B surface antigen (hpHBsAg). If antibodies to HBsAg (anti-HBs) are present in the serum, they will bind to the solid-phase antigen. In a second step, radiolabelled HBsAg ($^{125}$I-HBsAg) is added and reacts with the immobilized antibody. The amount of radiolabel bound to the solid phase, within limits, is in direct proportion to the amount of anti-HBs in the original specimen. An enzyme-linked immunoassay (EIA) version of this test (AUSAB EIA) has been available since 1983 and uses a biotinylated HBsAg-horseradish peroxidase (HRP) conjugated avidin mixture as a probe "detecting" reagent (I. Mushahwar, J. Virol. Methods, 16, 1 (1987)). In an improved version of this test, a biotin/antibiotin detection system has been substituted for the biotin/avidin detection reagent (I. Mushahwar et al., J. Virol. Methods, 16, 45 (1987)).

However, these assays detect primarily anti-S antibodies. They may not detect anti-PreS1 and anti-PreS2 antibodies, and certainly do not discriminate between samples which contain predominantly anti-PreS1, anti-PreS2 or anti-S antibodies. A need exists for assays which can detect both PreS1 and PreS2 proteins and antibodies against them, since several investigators have reported that antibodies to PreS1 and PreS2 are often the earliest antibody response to HBV infection in humans, may be linked to viral clearance, and may act as a neutralizing Ab. Such assays can be used to monitor vaccinees immunized with PreS-containing vaccines. For example, see Klinkert et al., J. Virol., 58, 522 (1986). Hess et al., Liver, 7, 245 (1987), have shown a relationship between HBV induced cytopathology and the presence of PreS1 staining in cells from liver biopsy, but the potential pathogenic role of PreS1 expression is unknown. Alberti et al., Hepatol., 7, 207 (1987), found that the presence of antibodies against PreS2 correlated with evidence of termination of viral replication and onset of convalescence. Therefore, the administration of PreS1 antibodies and/or PreS2 antibodies to exposed individuals or to HBV patients may be an important adjunct to current therapeutic regimens using hyperimmune globulin (HBIG). Since some HBIG preparations may comprise antibodies to PreS1 and/or PreS2, a need exists for an assay which can determine the amounts of these antibodies.

HBsAg is also antigenically complex, containing group-specific "a" determinants, defined as being present on all HBsAg particles, and two sets of what are normally mutually exclusive subtype-specific determinants, d/y and w/r. This gives four major subtypes of HBsAg which are designated adw, adr, ayw, and ayr. Additional determinants have also been described resulting in universal recognition of ten different HBsAg subtypes. For example, two of these subtypes are designated adyw and adywr, and represent instances where both "d" and "y" or "w" and "r" are found to exist in the same sample. The amino acid sequences for five distinct HBV subtypes of PreS2 are shown by A. R. Neurath et al., in Science, 224, 392 (1984)). However, the anti-PreS1 and anti-PreS2 mAbs which have been reported have not allowed discrimination of HBV subtypes, and it is unknown which changes in DNA sequence among HBV strains result in antigenic variation.

Mixed subtypes such as these presumably result from infection of cells with two or more viruses of separate genotype. The resulting mixed phenotype then occurs either as a mixtures of particles with different subtypes, or as particles which contain both subtypes. In the case where both subtype determinants occur on each particle, the mixed subtype particle is believed to result simply from phenotypic mixing, that is, from the intermixing of the two different subtypes of p25 and gp30 protein coded for by the two different viral S genes, upon assembly of the viral envelope. Particles composed of both forms of this protein would thus have both sets of subtype determinants. Upon viral transmission, particles with separate subtypes would be produced. See D. A. Paul et al., J. Virol. Methods, 13, 43 (1986). This complexity has led to a continuing need for methods to determine the HBsAg subtype of infected individuals. The ability to subtype HBV can help to track the route of HBV infection among populations and among individuals in cases of accidental and perinatal transmission.

## Summary of the Invention

The present invention provides two panels of monoclonal antibodies (mAbs) which define at least three distinct epitopes of the PreS1 protein and four distinct epitopes within the PreS2 protein of the HBV envelope. The isotypes and binding properties of six of these "groups" of mAbs is described in detail hereinbelow. The epitopal specificity for five of the groups is summarized on Table 1, below:

### Table 1
### Monoclonal Antibodies to PreS1 and PreS2 Proteins

| PreS1 | Epitope Includes:[a] | L Binding | M Binding | S Binding |
|---|---|---|---|---|
| Group 1 | 27-35 | + | - | - |
| Group 2 | 72-78 | + | - | - |
| | | | | |
| PreS2 | | | | |
| Group 1 | 120-145 | + | + | - |
| Group 2 | 123 (+ glycan) | - | + | - |
| Group 3 | 150-174 | - | + | - |

[a]  Amino acid position downstream from PreS1 methionine according to Figure 1 of A. R. Neurath et al., Cell, 46, 429 (1986).

These epitopes are specific to PreS1 or PreS2, e.g., they are retained after treatment of HBsAg-containing samples, such as Dane particles, which destroys essentially all of the antigenicity of the S-protein. Since these epitopes may be useful in HBV antibody assays, they are also within the scope of the invention, as well as their labelled forms. Some of the mAbs from each group exhibit "high affinity" binding ($K = 10^9$-$10^{12}$) to their epitopes. These mAbs represent preferred embodiments of this aspect of the invention.

As defined herein, an epitope comprising a region of the PreS1 or PreS2 protein is contained at least in part by the amino acid sequence of the region as set forth in Table 1, and may also include amino acid sequences flanking or adjacent to said region.

## Immunoassays

### 1. Antigen Assays

The present mAbs can be used to develop specific and sensitive immunoassays to detect HBV and HBsAg in physiological material such as human blood sera or plasma. If an HBV variant was so different from known variants that anti-S based detection systems, such as the Monoclonal Auszyme[R] assay, were unreactive, then the present anti-PreS based antigen detection assays could be used to screen the suspect specimens.

One assay which has been investigated using the present mAbs is a sandwich type radioimmunoassay (RIA) or enzyme-linked immunoassay (EIA) in which a liquid test sample comprising PreS1 or PreS2 protein, e.g., as the L and/or M protein, is reacted with a solid phase comprising a "capture antibody" for these proteins, which can be monoclonal or polyclonal. The capture antibody (Ab) can be one which binds to the S protein region, or one of the present mAbs, which binds specifically to an epitope in the PreS1 or PreS2 region. The captured PreS1 and/or PreS2 containing proteins are then detected and measured by reacting them with an anti-PreS1 or anti-PreS2 mAb of the present invention which has an attached detectable label, such as a radioisotope or an enzyme. Alternatively, the "detection antibody" can comprise a binding site for a detectable label, and the detectable label is added to the bound detection mAb in a separate step. The binding site for the detectable label can be an epitope which is bound by a third labelled antibody against the detection Ab. For example, enzyme-labelled anti-mouse antibody can be used to detect a bound murine mAb, such as those of the present invention. The amount of label bound to the captured PreS1-or PreS2-containing protein is then quantified, e.g., in counts per minute for a radiolabel or in absorbance (A) units for the color reaction catalyzed by an enzyme label, and correlated with standard PreS1- and PreS2-containing samples to yield a measure of the amount of PreS1- or PreS2-containing protein present in the test sample.

For example, the PreS1 antigen assays which have been developed using the present mAbs are summarized on Table 2, below.

## Table 2

| | Capture Ab on Solid Phase | Detection mAb |
|---|---|---|
| a) | Group 1 PreS1 | Group 2 PreS1 |
| b) | Polyclonal Auszyme[R]II (guinea pig; hyperimmune serum) | Group 1 PreS1 |
| c) | Group 1 PreS1 | Group 1 PreS1 |
| d) | Group 2 PreS1 | Group 2 PreS1 |
| e) | Auszyme[R] II (polyclonal) | Group 1 + Group 2 PreS1 |
| f) | Monoclonal Auszyme[R] (mAb against S protein) | Group 1 and/or Group 2 PreS1 |

PreS2 antigen assays have also been developed using Group 1, 2, and/or 3 mAb PreS2 in combination or alone with Auszyme[R] II or Monoclonal Auszyme[R] beads or with PreS2 mAb bound to the solid phase.

Useful solid phases include plastic beads, such as the polystyrene beads used in the Auszyme[R] assay; plastic microtiter plate wells and other plastic cell culture substrates, such as hollow fibers and sheets; woven or nonwoven fibrous solid phases such as those formed from paper, felt, or synthetic fibers, latex microparticles, porous ceramic beads such as silica, alumina or $ZrO_2$ beads and the like. The capture antibodies can be physically sorbed onto or into these substrates, or may be attached by covalent bonding methods known to the art.

The detection mAbs may be labelled by any of several techniques known to the art. A wide range of labelling techniques are disclosed in Feteanu et al., "Labeled Antibodies in Biology and Medicine", pages 214-309 (McGraw-Hill Int. Book Co., New York (1978)). The introduction of various metal radioisotopes may be accomplished according to the procedures of D. J. Hantowich et al., Science, 220, 613 (1983); Wagner et al., J. Nucl. Med., 20, 428 (1979); Sundberg et al., J. Med. Chem., 17, 1304 (1974); and Saha et al., J. Nucl. Med., 6, 542 (1976).

Among the radioisotopes used, x-ray-emitters, gamma-emitters, positron-emitters, and fluorescence-emitters are suitable for antibody detection. Preferred radioisotopes for labeling antibodies include Iodine 125, 131, or 123, Indium 111, Ruthenium 97, Copper 67, and Technicium 99. The halogens can be used more or less interchangeably.

One preferred labeling technique involves labeling with either Iodine-131 (I-131) or Iodine-125 (I-125) using an oxidative procedure wherein a mixture of radioactive potassium or sodium iodide and the antibody is treated with chloramine-T (N-chloro-p-toluene sulfonamide sodium salt) e.g., as reported by Greenwood et al., in Biochem. J., 89, 114 (1963) and modified by McConahey et al., in Int. Arch. Allergy Appln. Immunol., 29, 185 (1969).

In general, it is desirable to introduce as high a proportion of label as possible into the antibody molecule without destroying its immunospecificity. For each labelled antibody, the extent of the reduction in labeled antibody-antigen binding can be established by standard competitive cell binding assays. Preferably, at least about 30% of the initial binding activity of the antibody is preserved when 125-I or 131-I is used as the labels.

The present detection mAbs can be enzyme-labelled by direct covalent attachment of the enzyme or by use of a linkage such as the reaction between a biotinylated enzyme and an avidinated enzyme (see published European Patent Application No. 291,180, November 11, 1988). Useful enzyme labels include horseradish peroxidase (HRP), alkaline phosphatase, beta-galactosidase and glucose oxidase.

Because the present mAbs do not prevent the binding of target (M or L) protein to the solid phase, the present assays can also be carried in one step. In the one-step assay, the sample, the solid phase comprising the capture antibody and the detection mAb can be mixed together in the appropriate liquid phase. The amount of label bound to the solid phase is then detected as described hereinabove.

More specifically, one preferred two-step assay employs Auszyme[R]II beads as the capture Ab and an HRP-conjugated or $^{125}$I-PreS2 mAb of Group 1 or a Group 1/Group 2 mixture as the detection antibody. The sensitivity of this PreS2 assay was very high; about 1 ng PreS2 protein/ml total hpHBsAg or about 0.15 ng PreS2/ml of M protein can be detected. The assay was also specific for PreS2 to the extent that PreS2 protein can be detected in the presence of an 80,000-fold excess of experimentally-added S protein.

PreS1-containing L protein can be detected at $\leq$ 1 ng/ml sample without significant interference by added S protein, using capture Ab including a) Monoclonal Auszyme[R] beads, or b) Auszyme[R]II beads, and a detection mAb comprising (a) HRP-labelled Group 1 PreS1 mAb; b) HRP or $^{125}$I-labelled PreS1 Group 2 mAb, or c) $^{125}$I-labelled Group 1 and Group 2 PreS1 mAb as the detection antibody. Use of a PreS1 mAb-coated bead eliminates any interference with the assay by added S protein. The appearance and/or disappearance of PreS antigens in the blood of infected individuals has been reported to relate to the course of the disease and its prognosis, these assays may find clinical utility in the management of HBV. For example, assays which indicate only the presence or absence of these protein "markers," are not as useful as the present quantitative assays.

2. Antibody Assays

The present mAbs can be used as detection antibodies in competitive immunoassays which detect human antibody response to PreS1 and PreS2 regions on HBV. The preferred capture antigen is human plasma derived surface antigen (comprising S + M + L proteins) which is treated with a reducing agent (to cleave disulfide bonds) and iodoacetamide (to prevent reformation of disulfide bonds) to denature the S region. The denatured capture antigen is immobilized by adsorption onto a solid phase. The immobilized capture antigen is then combined with (a) a preselected amount of human blood serum or plasma which comprises endogenous HBV antigen (due to HBV infection) and (b) a preselected amount of a labelled PreS1-or PreS2-mAb of the invention. The solid phase is washed to free it of unbound material and the amount of the bound label is measured. The amount of bound, labelled mAb is inversely proportional to the amount of bound anti-HBV antibody. This assay provides a method to measure the concentration of anti-HBV antibody during the course of the disease, or to monitor antibody production in HBV vaccine recipients. This assay can also be used to measure endogenous or exogenous anti-PreS1 and anti-PreS2 antibodies in HBIG formulations or in vaccine recipients. The antibody which is bound by the capture antigen can also be measured by using a labelled PreS antigen of the invention as the detection antigen.

Other antigens useful as capture antigens in a competitive sandwich assay include a mammalian polyclonal antibody which binds to non-PreS epitopes on a fusion protein comprising PreS protein. Such non-PreS epitopes are found, for example, on CKS (3'-CMP-KDO synthetase). The polyclonal antibody to CKS is immobilized and mixed with seropositive serum or plasma and preselected amounts of (a) a fusion protein containing at least a part of the CKS enzyme and a PreS antigen and (b) a labelled PreS mAb. The immobilized antibody binds to the CKS region of the recombinant antigen fusion protein comprising part of the CKS enzyme and PreS protein.

The PreS region of the fusion protein provides a binding site which is competitively bound by endogenous anti-PreS antibody and by the added labelled PreS mAb of the invention. Thus, the strength of the signal due to the bound label which is detected is inversely proportional to the amount of the anti-PreS antibody in the sample. As in the case of the PreS1 and PreS2 assays described hereinabove, the serum and detection mAb can be added sequentially to the capture antigen or can be mixed with the capture antigen in one step.

Subtyping HBsAg

The present invention also provides a method to determine the specific subtype of HBV present in a physiological fluid such as human blood serum or plasma. To develop this method, samples containing HBV of the known subtypes are first contacted with samples of a monoclonal or polyclonal anti-S-antibody which has been bound to a solid support. Binding of S-containing proteins also captures PreS1 and PreS2 proteins, such as those in the L and M protein. The members of the subtype panel of bound proteins are then divided into two portions and reacted sequentially with pairs of PreS1 or PreS2 mAbs from the same or different groups, wherein the mAbs comprise a detectable label. Optionally, a plurality of dilutions of the sample are reacted with each mAb. The amount of the bound label is then quantified.

For selected pairs of mAbs, the relative amount of bound label in a series of samples of known subtype gives rise to patterns which are unique to a particular subtype. Since the ay and ad subtypes can be readily identified using a pair of known anti-S mAbs, "scanning" a known ay or ad HBsAg sample with pairs of the PreS1 or PreS2 mAbs can effectively complete the typing. For example, the ratio of label, e.g., in counts per minute, for two PreS2, Group 1 mAbs, 50-80-194 and 116-83-406 obtained following their reaction with a HBsAg of $ayw_1$ subtype, is more than 50 times that measured for any other HBV subtype. Ratio increases or decreases above average (taken as 1) of about 5-7 can be used to discriminate among subtypes.

Furthermore, the relative differences in the amounts of the present mAbs that can bind to a given amount of hepatitis B surface antigen provides a direct method for subtyping a physiological sample containing HBV. For example, the hepatitis B surface antigen in a sample are preferably first immobilized by contacting a first portion of the sample, such as blood plasma or serum, with a polyclonal antibody or a mAb against HBV surface antigen which is attached to a solid support. The first portion of the sample is then reacted with a first monoclonal antibody which binds to HBV PreS1 protein or HBV PreS2 protein, wherein said monoclonal antibody comprises a detectable label or a binding site for a detectable label. The anti-PreS1 or anti-PreS1 mAb is selected so that the amount bound indicates that at least one, and preferably that all but one, HBV subtype is not present. If necessary, these steps are then repeated to determine the amount of a Pre-S mAb of a different subclass bound to a second sample of immobilized HBV surface antigen, which in turn eliminates additional subtypes from consideration. Preferably, the relative amounts of bound, labelled mAbs determined after a given number of steps, preferably 1-3, will indicate which HBV subtype is present. As disclosed above, it is preferable to first identify the sample as ay or ad using known mAbs, but this is not necessary.

Also, one or more PreS1 and/or PreS2 mAbs can be immobilized, e.g., by spotting them onto discrete areas of a solid surface, such as a polymeric membrane, preferably along with an area of immobilized HBsAg as the positive control, and an irrelevant mAb as a negative control. Each area of PreS2 or PreS1 "capture" mAb is then exposed to an equal amount of a test sample comprising HBV, either by applying equal amounts of the sample to each capture mAb, or washing the surface comprising the capture mAbs with the sample, either diluted or neat. The binary complexes of HBsAg bound to each capture mAb are then detected by reacting the bound HBsAg with a monoclonal or polyclonal antibody reactive against the universal "a" determinant of HBV, which comprises a detectable label or a binding site for a detectable label, to yield a ternary complex. The amount of detection antibody bound to each area is then measured. The absolute amount (in the case of a single area of capture mAb) or the relative amounts (in the case of a plurality of areas of capture mAb) of bound detection antibody provides an indication of the partial or complete subtype of the HBV present in the test sample. Preferably, a plurality of areas of capture mAb are employed on a single surface, in close proximity, so that the resultant pattern of visualized or otherwise measured, bound detection mAb is indicative of the complete subtype of the HBV in the sample.

This represents the first use of mAbs which specifically bind the PreS1 or PreS2 proteins in HBV subtyping. The observed patterns of reactivity of the present mAbs with a larger number of individual plasma or sera samples can be used to develop an algorithm for their use as subtyping reagents as reported by Courouce et al., Develop. Biol. Standard, 54, 527 (1982) and J. R. Wands et al., PNAS USA, 81, 2737 (1984), in the case of S-specific mAbs.

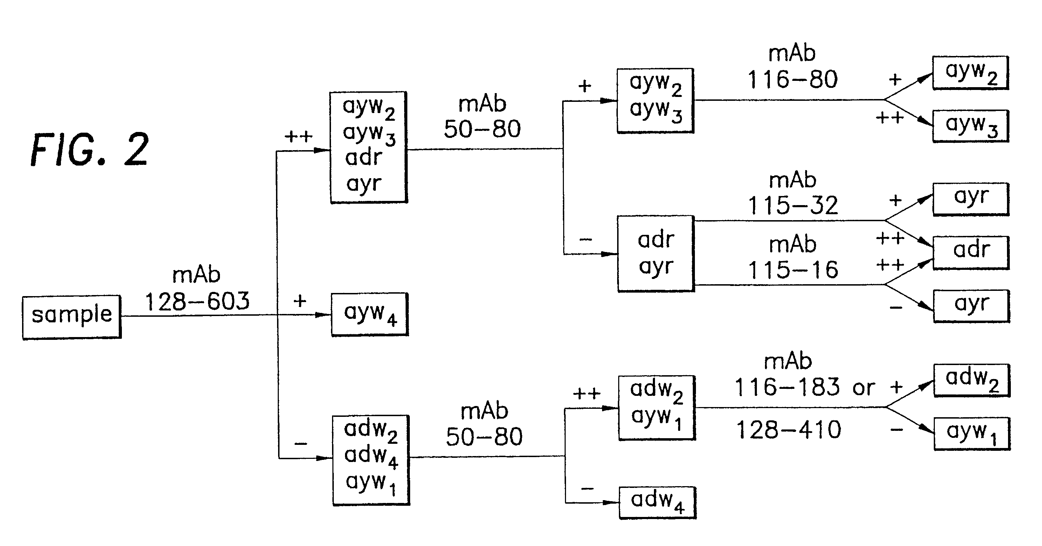

For example, with reference to Figure 2, the reactivity of the bound HBV surface antigens with a PreS2 group 3 mAb can type the HBV as $ayw_4$, or as one of the subtypes within two groups: (1)$ayw_2$, $ayw_3$, adr and ayr; or (2) as $adw_2$, $adw_4$ and $ayw_1$. Further reaction of a second sample of bound HBV surface antigens with a PreS2 group 1a mAb such as 50-80-194 can type the HBV as $adw_4$ or as one of the two subtypes within three groups of two subtypes each. A third sample of the bound HBV surface antigens can be prepared, and reacted with a third mAb, which will be selected based upon the reactivity of the first two samples, as depicted in Figure 2.

The mAbs of the present invention may be useful to more fully analyze the heterogeneity in the antigenic properties of HBV strains during horizontal transmission and in various population groups that would be missed with anti-S mAb-based subtyping methods. This is particularly true in view of the relatively small antigenic region of the S protein.

Vaccines and Monoclonal Antibody Therapy

At least some of the epitopes recognized by the present mAbs are immunodominant in the sense that endogenous antibody which competes with murine mAbs against these epitopes can be detected in seropositive individuals. These epitopes include the PreS2, Group 2 Asn 123 epitope listed on Table 1. Therefore, the present mAbs can be used to detect, characterize, and isolate epitopal sites on PreS1 and PreS2 that may be useful components of subunit HBV vaccines. These vaccines may also be useful to increase the titer of PreS1- and/or PreS2-antibodies in humans, so as to provide a source of HBIG which is enriched in these antibodies.

The present invention is also directed to a method for raising plasma immunogenicity to HBV by administering a pharmaceutical unit dosage form comprising one or more of the present antibodies to a patient who may be or has been exposed to HBV. Therefore, it is believed that the antibodies of the present invention will have therapeutic potential, either as a prophylactic agent to improve the prognosis of infected and/or diseased patients by retarding the clinical progression of the disease, or possibly, as a curative agent which can act to eliminate the virulence of the virus. The present antibodies might also represent a valuable adjunct to hepatitis B immune globulin (HBIG). HBIG therapy with supplemental PreS1 and/or PreS2 mAbs may be useful to prevent liver reinfection following liver transplant in HBV-infected individuals.

The isolated mAbs preferably are diluted with a pharmaceutically-acceptable liquid carrier, such as an aqueous IV fluid, prior to being assayed for bioactivity or administered as a unit dosage form in vivo. See Remington's Pharmaceutical Sciences, A. Osol, ed., Mack Pub. Co., Easton, PA (16th ed. 1980) at pages 1488-1496, the disclosure of which is incorporated by reference herein. The resultant solution is sterilized, e.g., by filtration. Preservatives commonly employed with IgG preparations, such as maltose, glycine or thimerosal, may be added in pharmaceutically-acceptable amounts.

The resulting solutions are preferably administered parenterally, e.g., by intravenous infusion or injection. The amount of mAb composition administered will vary widely, and will depend on the physique and physical condition of the HBV-infected patient. Such factors are necessarily empirical, and can be determined by the clinician, employing known HBV staging criteria. In some clinical situations, it may be necessary to administer a plurality of doses of the mAb composition, in order to neutralize the infectivity of viral particles as they are released from infected target cells.

Brief Description of the Drawing

Figure 1 is a schematic depiction of the protein composition of the HBV envelope.

Figure 2 is a schematic depiction of one embodiment of the HBV subtyping method of the invention.

Detailed Description of the Invention

The invention will be further described by reference to the following detailed examples wherein Protein A-Sepharose CL-4B, CNBr activated Sepharose CL-4B, Sepharose S-300 and all other chromatographic gels were from Pharmacia (Piscataway, NJ). Electrophoresis and Western blot reagents were obtained from Bio-Rad, Richmond, CA. Horseradish peroxidase (HRP) was from Toyoba, New York, NY. $Na^{125}I$ was obtained from Amersham, Arlington Heights, IL. The nine-member HBsAg subtype panel was from A-M Courouce (from the International Workshop of HBsAg Subtypes, Paris, April, 1975). Recombinant PreS1 (rPreS1) and recombinant PreS2 (rPreS2) were gifts from G. Okasinski (Abbott Laboratories, N. Chicago, IL). rPreS1 is a fusion protein which has a amino terminal portion of CMP-KDO synthetase (CKS) and a carboxy terminus containing PreS1 residue 12-120. The rPreS2 was a fusion protein with amino terminal portion of CKS and a carboxy terminus comprising PreS2 residues 123-175. Both rPreS1 and rPreS2 were HBV subtype adw2. Monoclonal antibodies (mAb) 18/1 and Q 19/10 were gifts from W. Gerlich (see K. H. Herrmann et al., J. Virol., 52, 396 (1984)).

Serum samples were tested with commercial RIA-EIA reagents supplied by Abbott Laboratories: Monoclonal Auszyme[R] and Ausria[R]II for HBsAg; Corab[R] for anti-HBc, Abbott HBe[R] for HBeAg and anti-HBe and Abbott Genostics[R] to detect HBV DNA.

To test for HBcAg in Dane preparations, samples (0.2 ml) were incubated with Monoclonal Auszyme[R] beads for 2 hrs at 40° C. Beads were washed with water, then incubated with 200 μl of 1% Tween 20 in Tris buffered saline for 30 minutes at 40° C. Beads were washed and incubated with solution containing Monoclonal anti-HBc conjugated to HRP. Beads were washed and incubated with the substrate solution described above.

Example 1 - Monoclonal Antibodies to PreS1 and PreS2

A. Dane Particle Purification

Human plasma with a high HBsAg titer was clarified by centrifugation at 2000 rpm for 20 minutes. The supernatant was centrifuged for 8 hr at 27,000 rpm in a SW28 rotor. The resulting pellet was resuspended in 0.01 M Tris, 1 mM EDTA, pH 7.6 (Buffer A) and this resuspension mixture was layered on 10% sucrose in buffer A which had been overlayered on a 2 ml 62% sucrose/Buffer A pad. After centrifugation for 8 hrs at 27,000 rpm in a SW28 rotor, fractions were collected and assayed for HBcAg. Peak fractions of HBcAg were pooled, diluted 1:4 in Buffer A, overlayed on a continuous 15-62% sucrose gradient, and spun for 24 hrs at 27,000 rpm. Fractions were collected and assayed for HBsAg, PreS2 Ag, PreS1 Ag, and HBcAg.

## B. Immunizations

Female 8-week old BALB/c mice were immunized with 10 $\mu$g of purified Dane particles emulsified in MP1 + TDM adjuvant (#R-700, RIBI Immunochem Research, Inc.) three times at three-week intervals. Serum samples were drawn two weeks after the last boost and titers were evaluated by enzyme-linked immunoassay (EIA) and Western blotting.

## C. Immunoassay for the Detection of Anti-Dane Antibody

Dane particles denatured in 5% beta-mercapto-ethanol (BME), 1% SDS and boiled for 3 minutes were diluted to 2 $\mu$g/ml in PBS and coated onto microplate wells during an overnight incubation at room temperature. Wells were washed with distilled water and blocked for 30 minutes with 200 $\mu$l of 3% BSA in PBS. Plates were washed and allowed to air dry. In the assay, the specimen was incubated 2 hr at room temperatures in the wells, incubation wells were washed, and HRP conjugated goat anti-mouse-Ig (#14-18-09, Kirkegaard & Perry Laboratories, Inc.) was added and incubated 1 hr. After the final wash, 100 $\mu$l of an OPD substrate solution (Abbott Laboratories) was added to each well. The color reaction was stopped by addition of 1 N sulfuric acid.

## D. Fusion Procedure

Responding mice were rested 2-4 months, then given a pre-fusion i.v. boost of 10 $\mu$g Dane particles in phosphate buffered saline (PBS). Three days later, the splenocytes were fused 1:1 with the SP2/0 myeloma line using standard protocols with slight modifications (G. Kohler et al., Nature, 256, 495 (1975)). The fusion pellet was briskly dispersed with 1 ml 50% PEG (ATCC NW 1450) for 1 minute, centrifuged in 20 ml media, and cells were resuspended in HAT-selective IMDM to be plated at $1.5 \times 10^5$ lymphocytes per well in 96-well tissue culture plates (#167008, NUNC). To promote hybrid survival, STM mitogen (#R-510, RIBI) was added into the initial plating media at a 1 to 200 dilution. The mitogen was not used in subsequent media changes and feedings.

## E. Establishment of Clones

Hybrid supernatants which were found positive on the anti-Dane screening EIA were evaluated by Western blotting to identify specific banding patterns. EIA and Western blot positive hybrids were cloned by limiting dilution. The clones selected for evaluation were derived from plates with approximately 10% growth per plate. Established clones were assayed as described above, grown up in T flasks and $1 \times 10^7$ cells/mouse were injected into pristane-primed BALB/c mice (Dominion Labs) to generate mAb containing ascites.

## F. Isotype Determination

MAb isotype was determined with the SBA Clonotyping System III kit (#5030, Southern Biotechnology Associates, Inc.) with slight modifications. EIA 96-well microplates were coated overnight at room temperature with 100 $\mu$l/well of a 1:1000 dilution of goat anti-mouse IgG+M (H+L) (Kirkegaard and Perry Laboratories, Inc.) as described for Dane particles above. Plates were washed and 50 $\mu$l of clone supernatants were added to appropriate wells for a 2 hr incubation at room temperature. Plates are washed and 100 $\mu$l/well of a 1:1000 dilution of the kit isotype specific conjugates were added to each sample for a 30-minute incubation. After the final wash, chromagen was added as described above.

## G. Monoclonal Antibody Purification

Antibody from IgG cloned lines was purified from mouse ascites fluid using a Affi-Gel Protein A MAPS II kit (Bio-Rad). Following equilibration of the column with binding buffer, ascites was mixed in a 1:1 ratio with binding buffer, passed through a 0.2 $\mu$m filter and loaded onto the column. After the sample was loaded onto the column, the 10 to 15 column volumes of binding buffer was passed through the column. IgG was then eluted with the supplied elution buffer, neutralized to pH 7.2, and dialyzed in PBS overnight at 4°C.

## H. Synthetic Peptides

Synthetic peptides corresponding to portions of the translational products of the PreS2 gene downstream from Met120 and the PreS1 gene downstream from Gly13 were made using an ABI solid phase peptide synthesizer (Applied Biosystems). Three peptides were constructed, then purified by high pressure liquid chromatography to yield PreS2: MQWNSTAFHQTLQDPRVRGLYLPAGG (120-145), TVNPVLTTASPLSSIFSRIGDPALN (150-174) and PreS1: GTNLSVPNPLGFFPDHQLDPAFGANSNNPDWDFNPVKDD (13-51), wherein single letter abbreviations for the amino acid residues are A, alanine; R, arginine, N, asparagine; D, aspartic acid; C, cysteine; Q, glutamine; E, glutamic acid; G, glycine; H, histidine; I, isoleucine; L, leucine; K, lysine; F, phenylalanine; M, methionine; P, proline; S, serine; T, threonine; W, tryptophan; Y, tyrosine; and V, valine.

## I. Peptide Inhibition of Mab Binding to Dane Particles

The synthetic peptides coding for PreS1 residues 13-51 and PreS2 residues 120-145 and 150-174 were used in inhibition assays. Microtiter EIA plates were coated by incubation of a PBS solution containing 2 $\mu$g/ml denatured Dane particles for 18 hrs at room temperature. Prior to the addition of the mAbs to the EIA plates, mAbs at 10 $\mu$g/ml were preincubated with 10 $\mu$g of either the 13-51, 120-145 or 150-174 peptide for 20 minutes. Fifty $\mu$l/well of sample was added to the plate, incubated for 20 minutes, and washed. A 1:1000 dilution of HRP-conjugated goat anti-mouse Ig (KPL) was added at 100 $\mu$l/well, incubated 30 minutes, washed, and color developed with o-phenylenediamine (OPD) substrate.

## J. Monoclonal Antibody Affinity

The antibody affinity was determined by the protocol of V. van Heyningen, Methods in Enzymology, J. J. Langone et al., eds., Academia Press, Inc. (1986) at page 472f. Antibodies were ranked as low ($K = 10^6$-$10^7$), intermediate ($K = 10^7$-$10^9$) and high affinity ($K = 10^9$-$10^{12}$). The affinity of mAbs in purified or unpurified culture supernatant was determined as the dilution factor or concentrations giving 50% maximal binding of the monoclonal antibody to denatured Dane particles. HRP conjugated goat anti-mouse Ig was used to detect bound antibody.

## K. Polyacrylamide Gel Electrophoresis/Western Blots

Analytical polyacrylamide gel electrophoresis (PAGE) was performed with a Bio-Rad slab gel apparatus with 12% running gel and 4% stacking gel. The gel and buffer formulations were those of U. K. Laemlli, Nature, 227, 680 (1970). Specimen was generally boiled for 5 minutes in a 1% SDS, 2.52% BME in Tris buffer. Western blotting was conducted essentially as described by H. Towbin et al., J. Immunol. Methods, 72, 313 (1984). After transfer of specimens onto nitrocellulose, nitrocellulose was soaked in blocking buffer (1% bovine hemoglobin, 0.1% v/v Tween 20 in PBS). Nitrocellulose was incubated with mAb to be tested at 0.5 to 1 $\mu$g/ml in blocking buffer for 1-2 hrs, then washed with phosphate-buffered saline (PBS), and incubated for 1-2 hrs with HRP conjugated goat anti-mouse IgG or IgM (0.5-1.0 $\mu$g/ml). Strips are washed, then developed with 4-chloro-1-naphthol/hydrogen peroxide substrate.

## L. Radioiodination and Enzyme Conjugation

MAb protein concentration was determined in mg/ml from the absorbance at 280 nm divided by the extinction coefficient, 1.38. Other protein concentrations were determined by the BCA (bichinchononic acid) procedure (Pierce Chemical, Rockford, IL) using bovine serum albumin as a standard. Purified mAb were radioiodinated using a chloramine T method to a specific activity of 20-30 $\mu$Ci/$\mu$g (Greenwood et al., Biochem. J., 89, 114 (1963)). Free $^{125}$I was separated from bound label by passage of the reaction mixture over a Sephadex G-50 column. MAb were conjugated to horseradish peroxidase (HRP) using the method of P. Nakane et al., J. Histochem Cytochem., 22, 1084 (1974). Conjugation ratios generally ranged from 1:1-1:4 mg/mg mAb:HRP.

## M. Competitive Binding to Dane Particles

Radioiodinated mAb was diluted into negative human plasma to produce a tracer solution at 0.2 μCi/ml. One hundred microliters of the tracer solution was added to 100 microliters of negative human plasma containing varying concentrations of mAb to be used for competition. A polystyrene bead coated with Dane particles at 2 μg/ml was added and incubated with this mixture for 2 hrs at 40°C or overnight at room temperature. The bead was washed with distilled water and counted for radioactivity.

N. Sandwich Antigen Immunoassays

Polystyrene beads (6 mm in diameter) were coated with capture antibody at a concentration of 20 μg/ml for 2 hrs at 40°C. Beads were rinsed with PBS, then incubated with a PBS solution containing 3% BSA for 1 hr at 40°C. Beads were rinsed with PBS containing 3% sucrose, then allowed to air dry.

Sample (0.2 ml) was incubated overnight at 20-25°C or for 2 hrs at 40°C with the antibody-coated bead. After washing with water, the bead was incubated with 0.2 ml of $^{125}$I labelled- or HRP-conjugated detection antibody for 2 hrs at 40°C. The bead was washed with water and counted for radioactivity in a gamma counter for the radioimmunoassay (RIA), or incubated with a HRP substrate solution (0.3 ml of 0.3% o-phenylenediamine-2-HCl in 0.1 M citrate-phosphate buffer (pH 5.5) containing 0.02% $H_2O_2$) for the enzyme-linked immunoassay (EIA). The enzymatic reaction was allowed to proceed for 30 minutes at room temperature, then stopped by the addition of 1 ml of 1 N $H_2SO_4$. Absorbance at 492 nm was measured using a Quantum II spectrophotometer (Abbott Laboratories).

O. Results

1. Purification of Dane Particles

Hepatitis B virions in human plasma were separated in pure form from the bulk of the 22 nm HBsAg particles and other plasma proteins by sucrose gradient centrifugation. Fractions were monitored by specific immunoassays for the detection of HBcAg, PreS1 Ag, HBsAg, and PreS1 Ag. The peak of S and PreS2 antigen activity co-sedimented with 22 nm particles, whereas PreS1 antigen and HBcAg co-sedimented as a significantly larger particle corresponding to 42 nm Dane particles. These data indicate that PreS1 and HBcAg, but not PreS2 and S could be used to help differentiate Danes from small 22 nm forms. Predominance of L protein (p39, gp41) in Dane particles as compared to purified HBsAg particles was observed by SDS PAGE as has been reported previously by Herrman et al., supra.

2. Immunization

Purified Dane particles (subtype ad; similar results can be obtained with ay) were used to immunize mice. Ten of ten Dane-immunized mice produced detectable anti-PreS1 and anti-PreS2 titers as measured by Western blotting and reactivity on the Dane coated bead immunoassay. Some immune response was elicited by inoculation with PreS2 synthetic peptide (120-145). Dane particles also proved to be significantly better immunogens in mice than a synthetic PreS1 peptide (12-53) inoculated as free peptide or coupled to KLH or BSA in adjuvant.

Hybridomas were initially screened by reactivity by solid phase EIA using Dane particles coated onto the solid phase and by Western blotting of Dane particles. Reactive clones were grown in ascites and mAb purified from ascites by protein A affinity chromatography.

3. PreS1 mAb

Purified mAb were characterized by Western blotting reactivity with purified Dane particles of ad and ay subtype, binding to synthetic peptides and recombinant PreS1 or PreS2 proteins, and reciprocal competition studies. Relative affinities were also determined as described in Materials and Methods. A summary of these results is shown in Table 3 for mAb PreS1.

TABLE 3

PRE-S1 MONOCLONAL ANTIBODIES

| Group | Clone Number | Isotype | Affinity | 13-51 Peptide Inhibition | Reactivity with rPre-S1 | Western Blot Reactivity Ad | Western Blot Reactivity Ay | PreS1 Region |
|---|---|---|---|---|---|---|---|---|
| 1 | 116-9-250 | IgG1 | 10E7-10E9 | + | + | + | + | 27-35 |
| | 116-11-193 | IgG2a | 10E6-10E7 | + | + | + | + | 27-35 |
| | 116-29-129 | IgG1 | 10E9-10E12 | + | + | + | + | 27-35 |
| | 116-80-179 | IgG1 | 10E9-10E12 | + | + | + | + | 27-35 |
| | 113-39-184 | IgG1 | 10E6-10E7 | + | + | + | + | 27-35 |
| 2 | 115-16-407 | IgG1 | 10E7-10E9 | - | + | + | + | 72-102 |
| | 116-8-151 | IgG1 | 10E9-10E12 | - | + | + | + | 72-102 |
| | 116-72-270 | IgG1 | 10E7-10E9 | - | + | + | + | 72-102 |
| | 116-109-364 | IgG1 | 10E7-10E9 | - | + | + | + | 72-102 |
| | 116-192-256 | IgG1 | 10E7-10E9 | - | + | + | + | 72-102 |
| | 116-86-293 | IgG1 | 10E9-10E12 | - | + | + | + | 72-102 |
| 3 | 116-7-188 | IgG1 | 10E7-10E9 | - | - | + | ND | ?-? |
| | 116-54-142 | IgG2a | 10E9-10E12 | - | - | + | - | ?-? |
| | 116-71-571 | IgG1 | 10E7-10E9 | - | - | + | - | ?-? |

ND = not done

The grouping of the PreS1 mAbs was determined from reciprocal competition immunoassays in which mAbs competed for binding to denatured Dane particle-coated beads. PreS1 mAbs could be segregated into three distinct groups from these studies. The group 1 and group 2 PreS1 mAbs bind to rPreS1 and also react with the L protein (p39 and gp42) of Dane particles (subtype ad and ay) by Western blotting. These mAbs do not react with HBsAg protein S (p24 and gp27) or with M proteins (gp33 and gp36). Group 1 and 2 mAbs do not compete for binding to Dane particle-coated beads. Group 1 mAbs specifically bind synthetic peptide (13-51) whereas Group 2 mAbs do not bind this peptide. The Group 1 mAbs also effectively compete with known anti-PreS1 mAb 18/7. Deletion mapping studies indicate that Group 1 mAbs bind to region 27-35 and Group 2 mAbs bind to region 72-102.

The Group 3 anti-PreS1 mAbs behave very differently from the other two groups. These mAbs react only with L protein by Western blotting for ad subtype but show no detectable reactivity for ay subtype. Group 3 did bind in a competable manner to Dane ad coated beads. These antibodies also did not react with any HBsAg subtypes in a sandwich immunoassay in which AUSZYME II beads were reacted with up to 45 $\mu$g/ml of HBsAg, then reacted with $^{125}$I-labelled mAb. Group 3 mAb showed virtually no binding to rPreS1 either in solid phase immunoassay or by Western blotting. One high affinity anti-PreS2 mAb (50-80-194) showed partial inhibition of the binding of all three groups of PreS1 mAb to Dane particles (21-34%) at high concentration. The Group 3 mAbs do not bind to peptide 13-51.

All other anti-PreS2 mAb and two anti-S mAb (H35 and H166) exhibited low or negligible inhibition of PreS1 mAb binding. H35 and H166 bind non-competing group-specific "a" determinants and were described previously by Peterson et al., J. Immunol., 132, 920 (1984). The observed partial inhibition results from steric constraints. H166 gave 25% inhibition of Group 1 PreS1 mAb binding but less than 10% for Groups 2 and 3.

### 4. PreS2 mAb

The PreS2 mAb could be divided into four groups based on reciprocal competition experiments. The profile of reactivity of these mAbs is summarized on Table 4, below.

TABLE 4

PRE-S2 MONOCLONAL ANTIBODIES

| Group | Clone Number | Isotype | Affinity | 120-145 Peptide Inhibition | Reactivity with rPre-S2 | Western Blot Reactivity Ad | Ay | Endo F Sensitive % Loss |
|---|---|---|---|---|---|---|---|---|
| 1a | 50-80-194 | IgG2a | 10E9-10E12 | + | + | gp33/36/39/42 | gp33/36/39/42 | 0 |
| | 116-183-406 | IgG1 | 10E7-10E9 | + | + | gp33/36/39/42 | gp33/36/39/42 | 0 |
| | 55-392-260 | IgG1 | 10E6-10E7 | + | + | gp33/36/39/42 | gp33/36/39/42 | 0 |
| 1b | 25-19-117 | IgG1 | 10E9-10E12 | + | + | gp33/36/39/42 | gp33/36/39/42 | 0 |
| | 128-410-194 | IgG2a | --- | + | + | gp33/36/39/42 | gp33/36/39/42 | --- |
| 2a | 116-34-263 | IgG1 | 10E9-10E12 | - | - | gp33/36 | gp33/36 | 100 |
| | 116-94-140 | IgG1 | 10E7-10E9 | - | - | gp33/36 | gp33/36 | nd |
| 2b | 115-32-181 | IgG1 | 10E7-10E9 | +/- | - | gp33/36 weak gp39/42 | gp33/36/ weak gp39/42 | 89 |
| 3 | 128-603-108* | IgG2b | | - | - | gp33/36 | gp33/36 | nd |
| 4 | 115-145-132 | IgG1 | 10E7-10E9 | - | - | gp39/42 | --- | nd |

*150-174 inhibition was +; all other mAbs were (-); 116-94 was not tested.

PreS2 group 1 mAb reacted with a linear epitope on both M and L proteins by Western blotting, to a synthetic peptide 120-145, and to a rPreS2 fusion protein containing residues 123-175.

The PreS2 Group 1 mAb may be further subdivided into Groups 1a and 1b based on their different reactivities with HBV subtypes. Group 1a and 1b mAbs bind distinct but closely spaced epitopes so that competition occurs by steric hindrance. This phenomenon is seen between known anti-S mAbs H95 and H35 (D. L. Peterson et al., J. Immunology, 132, 920 (1984)).

Group 2 and 3 PreS2 mAbs strongly bound only the M protein by Western blotting, but showed no reciprocal competition upon binding to immobilized Dane particles. The PreS2 group 2 mAbs were subdivided into groups 2a and 2b based on reciprocal competition studies. The group 2a mAb (116-34-263) completely blocked the binding of group 2b mAb (115-32-181) to Dane particle-coated beads, but group 2b mAb showed no ability to block group 2a mAb binding even at 120 $\mu$g/ml, which represented a 2000-fold excess of competing mAb. This differential inhibition did not result from differences in mAb affinity for Dane particles because some group 2a mAbs (e.g., 116-94-140) had equivalent or lower affinities than 115-32-181 (data not shown). Group 2a or 2b mAbs also differed in their reactivity to a synthetic peptide (120-145) and in their reactivity to L protein on Western blots.

Exclusive binding of group 2a mAb to M protein may be explained either by dependence upon a free PreS2 amino terminus or upon the carbohydrate moiety at asparagine 123. To determine the role of this glycan moiety in mAb binding, Dane particles were treated with a variety of glycosidases.

Treatment with the neuraminidase alone, neuraminidase + $\beta$-galactosidase, or neuraminidase + $\beta$-galactosidase + $\beta$-N-acetylglucosaminidase had little or no significant effect on group 2a reactivity or any other PreS2 mAb reactivity. Endoglycosidase F treatment completely abolished group 2a mAb (116-34-263) reactivity, but had no effect on group 1 mAb binding. The commercial endoglycosidase F contains two glycosidase activities: endo F and peptide-N-glycosidase F(PNGase F:E.C.3.5.1.52). Endo F, like endoglycosidase H (E.C. 3.2.1.96), cleaves the oligosaccharide chain between the di-N-acetylchitobiose moiety of some asparagine-linked glycans, primarily those of the high mannose type. PNGase F hydrolyzes at the glycosylamine linkage and generates a carbohydrate-free peptide chain. Since the endoglycosidase H had little or no effect on 116-34-263 binding to Dane particles, it is likely that the PNGase F activity in the commercial endoglycosidase F produced the cleavage resulting in loss of mAb binding. No attempt was made to distinguish which activity or whether the combination of enzyme activities was necessary to remove carbohydrate. The carbohydrate sequence of this PreS2 bound glycan has not been determined.

To evaluate the effect of endoglycosidase F treatment on the apparent molecular weight of M proteins after SDS-PAGE, treated and untreated Dane particles were analyzed by Western blot. The M protein bands of untreated Dane particles were strongly reactive for all PreS2 mAb. After treatment, no reactivity to Dane proteins was observed with group 2a mAb; however, bands at 28, 30, 35 and 40 kDa were reactive for group 1 mAb. The 28- and 30-kDa bands likely correspond to deglycosylated M protein. The appearance of higher molecular weight bands was unexpected, but may represent anomalous mobility of deglycosylated or aggregated M protein.

These data indicated that the presence of bound glycan was essential for Group 2a mAb reactivity with PreS2, but not for group 1 reactivity. Up to 90% of group 2b PreS2 mAb reactivity could be destroyed by endoglycosidase F treatment, but even after extended incubation of Dane particles some residual binding to deglycosylated M protein remained. The reactivity of 115-32-181 with deglycosylated M protein was confirmed by Western blot. Q 19/10, an anti-PreS2 mAb which reacts strongly with M protein (G. Gerken et al., Gastroenterology, 92, 1864 (1987)), also bound weakly to the deglycosylated M protein.

The group 3 PreS2 mAb (128-603-108) did not reciprocally compete with any other PreS2 mAb and was strongly reactive with M protein but unreactive with L protein of Dane particles (ad and ay) on Western blots. This mAb bound to a synthetic peptide (150-174) subtype ayw, but showed no reactivity with the 120-145 (adw2 sequence) peptide, with rHBsAg containing only the S gene produced in mouse L cells (L. Mimms et al., J. Virol. Methods, 25, 211 (1989)), or with rPreS2 produced in E. coli which contained residues 123-170 (sequence adw). This lack of 128-603 binding to the rPreS2 protein may be explained by its unique HBV subtype specificity, as described below.

Group 4 mAb showed no reciprocal inhibition with any other PreS2 mAb groups and reacted strongly to Dane ad subtype but not Dane ay subtype. These mAb were not reactive with rPreS2 or the 120-145 (adw2) peptide. This Mab reacts with PreS2 protein of type ayw1.

PreS1 mAb did not significantly inhibit any PreS2 mAb binding to Dane particles. However, PreS2 mAb, 50-80-194, at greater than 20 $\mu$g/ml showed partial inhibition of group 1 and 2 PreS1 mAb binding to Danes (21-34%). All other PreS2 mAb showed negligible (<10%) inhibition of PreS1 binding. Interestingly, 50-80-194 at very high concentration (>50 $\mu$g/ml) was able to inhibit 30-38% of group 2 PreS2 mAb binding. H166, an anti-S mAb, gave 25% inhibition of group 1 PreS1 binding but virtually no inhibition for group 2 PreS1 mAb. These data suggest a close proximity of the S, PreS2, and PreS1 epitopes on the surface of Dane particles.

Example 2. HBV Subtyping

Significant differences in the HBsAg subtype panel (Paris) reactivity were observed among mAb even within the same competition group. Pairwise comparisons of S, PreS2, or PreS1 mAb were performed to normalize binding within each region (Table 5).

TABLE 5

DIFFERENTIAL mAb REACTIVITY WITH PARIS SUBTYPE PANEL EXPRESSED AS SELECTED mAb BINDING RATIOS

| Ratios of mAb binding[a] | Paris subtypes | | | | | | | | | | |
|---|---|---|---|---|---|---|---|---|---|---|---|
| | ayw2 | ayw3 | ayw3* | ayw4 | ayw1 | adw2 | adrq+ | adrq- | ayr | adw4 | adyw |
| **S mAb** | | | | | | | | | | | |
| H95/H10 | 0.0 | 0.0 | 0.0 | 0.0 | 0.2 | 564.4 | 19.0 | 25.2 | 0.0 | 25.5 | 0.4 |
| **PreS2 mAb** | | | | | | | | | | | |
| 50-80/116-183[b] | 2.1 | 2.2 | 1.8 | 2.8 | 748.3 | 2.6 | 1.6 | 1.6 | 0.8 | 1.4 | 2.2 |
| 25-19/50-80 | 0.8 | 0.9 | 1.1 | 0.8 | 0.1 | 0.6 | 393.7 | 381.9 | 284.4 | 9.6 | 0.8 |
| 50-80/116-34 | 1.4 | 1.4 | 1.5 | 1.4 | 1.5 | 1.9 | 0.0 | 0.0 | 0.0 | 0.0 | 1.5 |
| 116-34/25-19 | 0.9 | 0.8 | 0.6 | 0.9 | 11.6 | 0.9 | 1.4 | 1.1 | 20.7 | 0.0 | 0.8 |
| 116-34/128-410 | 1.9 | 1.8 | 3.4 | 2.1 | 781.1 | 2.5 | 1949.5 | 1159.0 | 398.4 | 20.7 | 0.8 |
| 116-34/115-32 | 0.9 | 1.0 | 0.7 | 1.1 | 1.1 | 1.3 | 1.6 | 1.2 | 23.4 | 328.6 | 0.8 |
| 116-34/128-603 | 0.5 | 0.6 | 0.8 | 42.5 | 788.7 | 101.9 | 0.8 | 1.4 | 238.1 | 0.7 | 0.8 |
| **PreS1 mAb** | | | | | | | | | | | |
| 116-80/116-86 | 0.2 | 0.4 | 0.5 | 0.6 | 0.8 | 0.7 | 0.4 | 0.4 | 0.5 | 0.1 | 0.4 |
| 116-86/115-16 | 0.9 | 0.9 | 0.9 | 0.8 | 50.0 | 48.1 | 0.9 | 0.7 | 64.1 | 1.2 | 1.4 |

[a] To normalize binding, S/N value of S or PreS mAb binding to Paris subtype specimen was divided by the S/N value of another mAb binding within the same region. Values underlined are significantly different from those of ayw3 subtype. S/N values were determined as described under Materials and Methods.

[b] The last numeric designation, as shown on Tables 2-3, has been dropped for brevity.

Using binding ratios of anti-S mAb, ad subtypes could be easily distinguished from ay subtypes; adw2 distinguished from adr and adw4, and ayw1 differentiated from other ayw subtypes.

All Paris subtype members except *ayw₂* and *ayw₃* could be differentiated from one another on the basis of PreS2 mAb reactivity (Table 6).

TABLE 6

SUMMARY OF RELATIVE REACTIVITY OF PreS2 and PreS1 mAb WITH THE PARIS SUBTYPE PANEL (1975)

| Group | Clone no. | $ayw_2$ | $ayw_3$ | $ayw_4$ | $ayw_1$ | $adw_2$ | $adr$ | $ayr$ | $adw_4$ |
|---|---|---|---|---|---|---|---|---|---|
| PreS2 mAb[a] | | | | | | | | | |
| 1a | 50-80-194 | ++ | ++ | ++ | ++ | ++ | - | - | - |
| 1a | 116-183-406 | ++ | ++ | ++ | - | - | - | - | + |
| 1b | 25-19-117 | ++ | ++ | ++ | + | ++ | - | ++ | - |
| | 128-410-194 | ++ | ++ | ++ | - | + | ++ | - | ++ |
| 2a | 116-34-263 | ++ | ++ | ++ | ++ | ++ | ++ | ++ | ++ |
| 2b | 115-32-181 | ++ | ++ | ++ | ++ | ++ | + | + | - |
| 3 | 128-603-108 | ++ | ++ | + | - | - | ++ | ++ | - |
| PreS1 mAb[b] | | | | | | | | | |
| 1 | 116-80-179 | + | ++ | ++ | ++ | ++ | ++ | ++ | - |
| 2 | 116-86-293 | ++ | ++ | ++ | ++ | ++ | ++ | ++ | ++ |
| | 115-16-407 | ++ | ++ | ++ | - | - | ++ | - | ++ |

[a] ++, S/N > 100, +, 5 < S/N < 75; -, S/N < 5; S/N is cpm of sample-containing HBsAg divided by cpm of negative human plasma.

[b] ++, S/N > 30; +, 5 < S/N < 30; -, S/N < 5.

In group 1 PreS2 mAb, 50-80-194 and 116-183-406 gave little or no reactivity with subtypes *ayr*, *adw₄*, or *adr*; however, their binding to *ayw₁* subtype differed significantly. 50-80-194 binds strongly to *ayw₁* but

17

116-183-406 shows virtually no binding to this subtype (Tables 5-6).

Another group 1 mAb, 128-410-194, is similar to 116-183-406 in reactivity for all subtypes except $adw_2$. In fact, 128-410-194 binds with high affinity only to $ayw_{2-4}$ subtypes. A fourth group 1 mAb, 25-19-117, binds to all Paris subtype members, but showed a significantly lower affinity for subtypes $ayw_1$ and $adw_4$.

In contrast to the group 1 mAb binding, group 2a mAb (116-34-263) shows high affinity binding to all Paris subtypes. Comparison of group 2a mAb (116-34-263) and group 2 mAb (115-32-181) showed that the $ayr$ subtype was significantly more reactive to group 2a, although both 115-32-181 and 116-34-263 binding is dependent upon glycan at asparagine 123. Thus, a PreS2 group 2 mAb is preferred as a detection antibody in sandwich-type immunoassays, as it is capable of binding substantially equally to all of the HBV subtypes.

The group 3 PreS2 mAb 128-603-108, gave subtype reactivity unlike any other PreS2 mAb. This mAb does not show significant binding to subtypes $adw_2$, $adw_4$, or $ayw_1$ and has low affinity for $ayw_4$ compared to $ayw_2$, $ayw_3$, $ayr$, and $adr$ (Table 6).

Since the PreS1 region contains no bound carbohydrate, all mapped epitopes are defined by amino acid sequence alone. The most striking subtype difference is observed between two group 2 PreS1 mAb, 116-86-293 and 115-16-407. 115-16-407 binds weakly to $ayw_1$, $ayr$ and $adw_2$ compared to 116-86-293. The simplest explanation for this reactivity difference would be one amino acid change in this epitope common to all three subtypes. Another interesting comparison can be made between the reactivity of PreS1 group 2 (116-86-293) and group 1 (116-80-179) mAb. The ratio of 116-86-293 to 116-80-179 binding is greater for the $adw_4$ subtype than the ratio for all other subtypes (Table 5). Two explanations exist: the group 1 epitope which is nearer to the amino terminus of PreS1 region than the group 2 epitope has been preferentially lost or destroyed (e.g., by proteolysis) or $adw_4$ has a unique sequence in the group 1 PreS1 region. Several PreS1 mAb from different hybridomas show little or only subtle changes in reactivity with Paris HBsAg subtypes, suggesting that the PreS1 group 1 and group 2 epitopes are relatively conserved.

In addition to the eight major subtypes in Table 6, three other subtypes were identified in Paris (1975). One termed $adw_3^*$ was indistinguishable from $ayw_2$ and $ayw_3$ on the basis of S and PreS mAb binding (Table 5). Another subtype, designated $adrq$-, showed almost identical reactivity to $adr$ (alternatively designated as $adrq$+ by Courouce-Pauty et al, Vox Sang., 44, 197 (1983))(Table 5). Subtle differences between these subtypes were previously based on differential reactivity with polyclonal or affinity absorbed polyclonal antisera. The Paris subtype designated $adyw$ was from a patient in which the $ad$ and $ay$ subtypes occur on separate particles. (D. Paul et al., J. Virol. Methods, 13, 43 (1986)). S and PreS mAb reactivity was consistent with a mixture of $adw$ and $ayw$ particles, but no attempt was made to separate ad from $ay$ particles which would have allowed detailed characterization of the individual subtypes.

From these data, it is possible to determine the HBsAg subtype of various infected individuals. Subtypes d vs. y may first be distinguished by measuring serum HBsAg reactivity to the known anti-S mAbs, H95 and H10 which react strongly with d or y subtype, respectively. Alternatively, a subtyping method using exclusively the PreS1 and PreS2 mAbs of the invention can be used. A representative subtyping scheme is depicted in Figure 2, wherein the third number has been dropped from the mAb designation for brevity. Because of the large difference in HBV subtype reactivity observed with the present mAbs, generation of detailed binding curves using S-specific mAb is not necessary.

In another embodiment of the subtyping method, a plurality of monoclonal antibodies (mAb) against HBV PreS1 and PreS2 proteins are spotted at individual locations on a solid phase so that simultaneous determinations from a single specimen are possible. A preferred solid phase is a nitrocellulose membrane that has been embossed by an ultrasonic horn to form multiple isolated "islands." Typically, the pattern employs thirteen 2.5 mm diameter circles to which the capture antibody is applied. Capture mAb are spotted onto the nitrocellulose "islands" and dried. To prevent nonspecific binding, the reaction surface is preferably overcoated in a blocking step with BSA or gelatin. The solid phase is then assembled into a plastic test card. Up to 30 separate mAb (or mAb applied at different concentrations) can be applied to a single reaction surface.

A battery of 13 mAb; 3 against the S region (H95, H166, H10), 7 against the PreS2 region (50-80-194, 25-19-117, 116-183-406, 128-410-194, 116-34-263, 115-32-181, 128-603-108) and 3 against the PreS1 region (116-80-179, 116-86-293, 115-16-407) is particularly useful for examining HBV subtype variability in the envelope region. A spot of HBsAg and spot with an irrelevant mAb as positive and negative control, respectively, is also included.

In the assay, specimen (neat or diluted into diluent) is applied to the reaction surface and agitated at 35-40°C for 1-18 hrs. The reaction surface is washed with buffer solution, then enzyme-labeled monoclonal or polyclonal antibody (conjugate) reactive against universal a determinant of HBV is added and allowed to incubate for 1-2 hrs. Conjugate is aspirated, the reaction surface is washed again, and a solution containing a precipitating substrate for the conjugate is added. To stop the reaction, the reaction surface is washed with buffer solution and allowed to dry. Color in the individual spots may be scored visually or quantified with a front face reflectometer.

Example 3. PreS1 and PreS2 Antigen Assays

A. PreS2 Antigen Assay

Specifically for the PreS2 antigen assays, beads coated with goat anti-HBs (Abbott Laboratories) were used with radio- or enzyme-labelled mAb (50-80-194 or 25-19-117). Most studies were conducted using Auszyme$^R$II beads (Abbott Laboratories) comprising bound polyclonal, guinea pig anti-S antibodies, and a tracer solution containing a mix of $^{125}$I labelled mAb anti-PreS2 (Group 2: 116-34-263 and/or Group 1: 50-80-194).

B. PreS1 Assay

To detect PreS1 antigen, monoclonal Auszyme$^R$ or Auszyme$^R$II beads were used with radio- or enzyme-labelled mAb 116-29-129 (Group 1). Also, beads coated with mAb 116-80-179 (Group 1) were used with radio- or enzyme-labelled 116-8-151 or 116-72-270 (both Group 2), as the probe.

C. Results

1. PreS2 Antigen Assays

A three-step configuration using goat anti-HBs antibody (Abbott Laboratories) coated beads and PreS2 mAb (25-19-117) (Group 1b) in a second step followed by a detection step using HRP conjugated goat anti-mouse IgG was used for many experiments and gave good sensitivity and selectivity. A two-step procedure with equivalent selectivity and sensitivity has also developed with the Auszyme$^R$II beads and using an HRP conjugated or $^{125}$I-PreS2 mAb [25-19-117 (1b), 50-80-194 (1a) + 116-34-263 (Group 2a)] as the detection mAbs. One hundred specimens testing negative for HBsAg by the monoclonal Auszyme$^R$ were all unreactive in the PreS2 antigen assays described above. Based on these negative populations, a cutoff for the EIA assay of 0.05 + NCx or 4XNCx for the RIA were chosen (greater than 7 standard deviations from the negative population mean). To quantify PreS2 Ag, dilutions of a hpHBsAg preparation were run in each assay. The percent of total M protein contained in hpHBsAg preparations was determined by scanning densitometry of Coomassie stained SDS gels. The preparation used for these quantitation experiments contained 15% M protein. Sensitivity of the PreS2 assay was determined to be approximately 1 ng/ml of total hpHBsAg or 0.15 ng/ml of M protein.

Recombinant HBsAg containing only the S gene at concentrations of up to 300 μg/ml did not inhibit the anti-PreS2 assay indicating that PreS2 Ag can be detected in the presence of a 80,000-fold excess of S protein. Two hundred plasma specimens from HBV carriers were quantitatively assayed for HBsAg using the monoclonal Auszyme$^R$ assay and for M protein using the PreS2 Ag assays described above. Ninety-nine percent (102/103) of HBeAg positive carriers and 95% (81/85) of anti-HBe positives were reactive by the PreS2 antigen assay. On average, the levels of M proteins are 11-fold higher (and those of HBsAg are 5-fold higher) in the HBeAg positive carriers compared to the anti-HBe positive group.

Specimens with M protein concentrations higher than 10 μg/ml fall exclusively into the HBeAg positive group. There is extensive overlap in M protein concentrations between HBeAg and anti-HBe positive specimens below these values. The HBV DNA concentrations in a subset of these populations was determined and showed poor correlation with M protein (data not shown).

These data taken together suggest that PreS2 antigen does not provide any additional information beyond that obtained from an anti-S specific test (monoclonal Auszyme$^R$) and does not serve as an accurate indicator of active viral replication.

2. PreS1 Antigen Assay

Four sandwich immunoassays were developed for detection of PreS1 Ag: one used a monoclonal Auszyme$^R$ bead and HRP conjugated PreS1 mAb (116-29-129, Group 1) as detection system; the second used PreS1 mAb (116-80-179, Group 1) as the solid phase capture Ab and HRP conjugated or radioiodinated PreS1 mAb (116-72-270; Group 3) as the detection mAb; the third utilized Auszyme$^R$ II beads and tracer

containing $^{125}$I-labelled 116-29-129 and 116-8-151 (Group 2) as the detection mAb; and a fourth utilized Auszyme[R] II beads and $^{125}$I-labelled 116-80-179 (Group 1) and 116-86-290 (Group 2).

To quantify PreS1 Ag, dilutions of purified Dane particles were run on each assay. The percent of total L protein contained in the Dane preparation was determined similarly to the procedure used for M protein. The Dane preparation used for quantitation was about 10% L protein. To determine an assay cutoff, 100 HBsAg negative sera and plasma were run in the assay. All were unreactive when a cutoff of 0.05 + NCx in the EIA or 4 times NCx in the RIA (greater than seven standard deviations from the negative population mean) was used.

All HBeAg positive specimens from HBV carriers (n = 229) and 96.6% (57/59) of anti-HBe positive specimens were reactive in the PreS1 Ag assay. PreS1 Ag concentration varied over a wide range from less than 8 ng/ml to greater than 16 μg/ml. No anti-HBe positive specimens had greater than 0.9 μg/ml L antigen compared to 37.5% of the HBeAg positive specimens. Significant overlap in PreS1 Ag concentrations in sera is observed between HBeAg and anti-HBe reactive specimens at L protein concentration in sera less than 0.9 μg/ml.

Ninety-three percent of HBeAg positive specimens had detectable HBV DNA in their sera (mean [HBV-DNA] = 63.8 pg/ml, range 0.5 - 445 pg/ml) compared to 20% of anti-HBe positive specimens (mean [HBV-DNA] = 2.14 pg/ml range 1.7 - 2.4 pg/ml).

Sera with high HBV DNA concentrations tended to have higher concentrations of PreS1 Ag. There was a significant quantitative correlation between these markers (R = 0.52). A lower quantitative correlation was observed between PreS1 Ag and HBsAg concentrations (R = 0.38).

Example 4. Antibody Assays

To perform an assay for anti-PreS2 antibodies in the blood of seropositive individuals, 200 μl of serum was added to a single 0.25 inch polystyrene bead coated with HBsAg comprising reduced and iodoacetamide-capped S protein. The mixture was incubated for 18 hrs at 25° C; then washed with water and 200 μl of $^{125}$I-labelled mAb 50-80-194 added (1 μC/ml) in a diluent containing animal sera. The mixture was incubated for 2.0 hrs at 40° C, washed to remove free mAb, and the bead was counted. The time course HBsAg, anti-HBs antibody and anti-PreS2 antibody for one HBV patient is summarized on Table 7, below.

## Table 7

| Patient's Blood Sampled on Day | Auszyme[R] HBsAg | Ausab[R] (anti-HBs) | Competitive Anti-PreS Ab S/CO* | Interpretation |
|---|---|---|---|---|
| 1 | − | − | 1.624 | − |
| 31 | + | − | 1.620 | − |
| 36 | + | − | 1.564 | − |
| 44 | − | + | 0.968 | + |
| 52 | − | + | 0.982 | + |
| 62 | − | + | 0.858 | + |
| 93 | − | + | 0.192 | + |
| 122 | − | + | 0.166 | ++ |

* Ratio of cpm (sample)/cpm(cut-off); negative control value = 2; if S/CO < 1.0, then sample is considered positive (+) for antibody against PreS2 protein.

Discussion

20

MAb were generated which defined at least three distinct epitopes in the PreS1 region and four distinct epitopes within the PreS2 region of the HBV envelope. As observed by Western blotting, all of these patterns are retained after treatment of Dane particles by heat, SDS, and B-mercaptoethanol (BME). These treatments destroy most of the antigenic sites on the S protein. Determinants may be linear epitopes or are conformational ones which can reform after transfer of protein to nitrocellulose. No cysteines are present in PreS1 and PreS2 regions so that proper reforming of disulfide bonds is not necessary to retain the native conformation. This is in distinct contrast to most antigenic epitopes of S (p24 and gp27) which are highly dependent on disulfide bond formation and proper assembly into particles. Most of the S determinants are destroyed during the SDS/BME denaturation step during sample preparation for Western blotting.

All mAb were also tested for reactivity with native Dane particles and, therefore, all epitopes defined by these mAbs must be exposed on the viral surface. The data demonstrate that for one group of anti-PreS2 mAb (Group 2), the glycan at asparagine (Asn) 123 in the PreS2 region comprises an essential part of the determinant. Because this mAb does not bind to other human serum glycans, it is likely that the antigenic determinant consists of both carbohydrate and protein as has been demonstrated for the human blood group protein, glycophorin A (MN antigen).

The present PreS2 and PreS1 mAbs have proven useful in developing specific and sensitive immunoassays to detect M and L protein in human sera and plasma. Previous reports claimed an absolute correlation between the presence of serum markers for HBeAg and the presence of M protein (M. Imai et al., Gastroent., 76, 242 (1979)). Another claim in the literature was that the presence or absence of PreS1 Ag correlates with infectivity and the presence of HBV DNA. The data support the work of Hu et al. (cite), which showed a lack of qualitative correlation with the HBeAg status, although HBeAg individuals have a higher average concentration of HBsAg, PreS2 Ag, PreS1 Ag and HBV DNA.

Virtually all HBsAg carriers were found to have detectable PreS1 and PreS2 Ag in their sera or plasma when the present, sensitive immunoassay is used for detection. It is clear then that this PreS Ag assay gives similar diagnostic and screening information to that from an S protein (HBsAg) specific test (Monoclonal Auszyme$^R$). Quantitation of HBsAg, PreS1, and PreS2 antigen does give some additional information, in that individuals with S, M, and L protein concentrations above the threshold values of 100 $\mu$g/ml, 10 $\mu$g/ml and 0.8 $\mu$g/ml, respectively, were HBeAg positive and HBV DNA positive (sens = 0.5 pg/ml). However, for the majority of HBV-infected individuals with hepatitis B envelope antigen concentrations below these threshold values, anti-HBe/HBeAg status or HBV DNA detectability could not be accurately predicted.

Other uses for high affinity PreS2 and PreS1 mAbs include mapping the hepatocyte binding region of the L protein, as well as intracellular and cell surface staining of HBV infected hepatocytes and experimentally transfected hepatoma cell lines. Lower-affinity PreS1 and PreS2 mAbs of the invention have been successfully used to affinity purify recombinant and plasma derived PreS2 and PreS1 containing proteins at high yields.

Samples of certain of the hybridoma lines producing monoclonal antibodies of the invention have been deposited in accord with the Budapest Treaty in the International Recognition of the Deposit of Microorganisms for the Purposes of Patent Procedure at the American Type Culture Collection (ATCC), 12301 Parklawn Drive, Rockville, Maryland 20852 and have received the designations shown on Table 8, below.

## Table 8

| Hybridoma | ATCC Designation |
| --- | --- |
| HBV 116-29-129 | HB 10118 |
| HBV 116-8-151 | HB 10119 |
| HBV 116-86-293 | HB 10120 |
| HBV 50-80-194 | HB 10121 |
| HBV 116-34-263 | HB 10122 |
| HBV 128-603-108 | HB 10445 |
| HBV 115-32-181 | HB 10444 |
| HBV 128-410-194 | HB 10443 |

The invention has been described with reference to various specific and preferred embodiments and techniques. However, it should be understood that many variations and modifications may be made while remaining within the spirit and scope of the invention.

## Claims

1. A monoclonal antibody which binds to an epitope in the region of the hepatitis B virus (HBV) PreS2 protein comprising the amino acid sequence:
   TVNPVLTTASPLSSIFSRIGDPALN
   wherein said monoclonal antibody binds to the HBV M protein, but not to the HBV S protein or the HBV L protein.

2. The monoclonal antibody of claim 1 which further comprises a detectable amount of a bound label comprising a radioisotope or an enzyme, and wherein the radioisotope is a radioactive halogen atom.

3. A method to subtype a hepatitis B virus (HBV) which comprises the steps of:
   (a) reacting a first portion of a sample of an immobilized hepatitis B surface antigen with a first monoclonal antibody which binds to HBV PreS1 protein or HBV PreS2 protein; wherein said monoclonal antibody comprises a detectable label; and
   (b) calculating the amount of bound labelled first monoclonal antibody, wherein said amount indicates that at least one HBV subtype is not present.

4. The method of claim 3 further comprising repeating steps (a) and (b) using a second portion of the sample and a second labelled monoclonal antibody which binds to a second HBV PreS1 protein or a second HBV PreS2 protein, wherein said second monoclonal antibody comprises a detectable label, so that the amount of bound labelled second monoclonal antibody indicates that at least one additional HBV subtype is not present.

5. The method of claim 4 further comprising repeating steps (a) and (b) using a third portion of the sample and a third monoclonal antibody which binds to a third HBV PreS1 protein or a third HBV PreS2 protein, so that the amount of labelled third monoclonal antibody indicates that all but one HBV subtype is absent.

6. The method of claim 3, 4 or 5 wherein the monoclonal antibodies are each radiolabelled or enzyme-labelled.

7. The method of claim 3, 4 or 5 wherein the hepatitis B surface antigen is immobilized by binding it to a polyclonal antibody against hepatitis B surface antigen which polyclonal antibody is immobilized to a solid support.

8. A method to subtype a hepatitis B virus (HBV) which comprises the steps of:
   (a) immobilizing a plurality of monoclonal antibodies, individually in a discrete area on a solid surface, wherein each of the monoclonal antibodies binds to a different HBV PreS1 or HBV PreS2 epitope;
   (b) contacting each of the discrete areas of monoclonal antibody with an equal portion of a sample comprising HBV to form a binary complex between at least some of said plurality of said monoclonal antibodies and said HBV; and
   (c) contacting each of said discrete areas with an antibody which binds to the a determinant of HBV, wherein said antibody comprises a detectable label, to form a ternary complex between said labelled antibody and said binary complex; and
   (d) measuring the amount of label bound to each area, wherein the relative amounts of label bound to each area is indicative of the complete subtype of the HBV in the sample.

9. The method of claim 8 wherein said plurality of monoclonal antibodies comprises at least one monoclonal antibody which binds to a HBV PreS1 epitope and at least one monoclonal antibody which binds to a PreS2 epitope.

# FIG.1

Schematic Representation of HBV Envelope Protein

GP42 / P39 — 226AA — $G_c$ — L-protein

108-119AA / 55AA

GP36 / GP33 — $G_c$ / $G_m$ — M-protein

GP27 / P24 — $G_c$ — S-protein

PreS1 — PreS2 — S

1 — 120 — 174 — 400 (AA Scale)

FIG. 2

EP 0 456 215 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | EP-A-0 389 983   (ABBOTT LABORATORIES)<br>* Page 3, line 50 - page 7, line 35 *<br>– – – | 1 | C 12 P 21/08<br>G 01 N 33/577<br>G 01 N 33/576 |
| A | FR-A-2 637 184   (INSTITUT NATIONAL DE LA SANTE ET DE LA RECHERCHE MEDICALE)<br>– – – – – | | |

|  |
|---|
| TECHNICAL FIELDS SEARCHED (Int. Cl.5) |
| C 07 K<br>C 12 P |

| The present search report has been drawn up for all claims | | |
|---|---|---|
| Place of search | Date of completion of search | Examiner |
| The Hague | 02 August 91 | REMPP G.L.E. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document